# EUROPEAN PATENT APPLICATION

(11) **EP 4 573 926 A1**
(43) Date of publication of application: **25.06.2025**
(21) Application number: 22948189.0
(22) Date of filing: 19.08.2022
(51) Int. Cl.: A23L 33/18, A23L 33/175, A61K 38/16, A61P 25/16, A23L 2/38, A23P 10/28, A23P 10/30

(54) **COMPOSITION CONTAINING OSMOTIN PROTEIN AS ACTIVE INGREDIENT FOR PREVENTION, ALLEVIATION, OR TREATMENT OF PARKINSON'S DISEASE**

(71) Applicant: Industry-Academic Cooperation Foundation Gyeongsang National University, Jinju-si, Gyeongsangnam-do 52828 (KR)
(72) Inventor: KIM, Myeong Ok, Jinju-si, Gyeongsangnam-do 52819 (KR); PARK, Jun Sung, Jinju-si, Gyeongsangnam-do 52860 (KR); CHOE, Kyonghwan, Seoul 08275 (KR); PARK, Tae Ju, Jinju-si, Gyeongsangnam-do 52819 (KR)
(74) Representative: Ipsilon NNY
(86) International application number: PCT/KR2022/012416
(87) International publication number: WO 2024/038934

(57) **Abstract**

The present invention relates to a composition for preventing, ameliorating, or treating Parkinson's disease comprising osmotin protein as effective component, and, by having the effects of alleviating behavior and motor skill deficits induced by MPTP/α-synuclein, protecting dopaminergic neurons, regulating the expression level of neuroinflammation-related proteins, inhibiting apoptotic cell death induced by MPTP/α-synuclein, alleviating cell damage caused by overexpression of α-synuclein (A53T), reducing the accumulation of α-synuclein caused by activation of AMPK and subsequent autophagy, regulating the dendritic complex structure, increasing the spine density in pyramidal neurons, alleviating the cognitive deficits, and restoring the expression of synaptic markers, the osmotin protein can be advantageously used for a functional health food or a pharmaceutical product for preventing, ameliorating, or treating Parkinson's disease.

## Description

### TECHNICAL FIELD

The present invention relates to a composition for preventing, ameliorating, or treating Parkinson's disease comprising osmotin protein as effective component.

### BACKGROUND ART

Degenerative neurological disorders refer to the progressive structural and functional loss of nerve cells (neurons). These disorders primarily affect specific areas of the nervous system, leading to symptoms such as dementia, extrapyramidal abnormalities, cerebellar dysfunction, sensory impairment, or motor dysfunction. Multiple regions can be also simultaneously affected by the disorders, resulting in complex symptoms. A diagnosis is usually made based on the clinical manifestations shown by patients. However, the diagnosis is often challenging due to the diverse and overlapping clinical symptoms exhibited by patients who are suffering from different disorders.

Parkinson's disease as one of the many neurological disorders is characterized by the gradual destruction and loss of dopamine-producing neurons in the substantia nigra (SNc) of a brain. This leads to a significant decrease in dopamine level in the striatum. The disease is a chronic, progressive, and degenerative disorder of the nervous system and characterized by distinctive symptoms including resting tremors, rigidity, bradykinesia (slow movements), and postural instability. It is estimated that around 1% of the population over the age of 60 is affected by Parkinson's disease.

Meanwhile, osmotin derived from plants is involved in the regulation of fatty acid oxidation, glucose acquisition, phosphorylation (AMP kinase), and signal transduction pathways. Osmotin (24 kDa) is a stable protein belonging to the PR-5 family, sharing homology with the sweet-testing protein thaumatin. It is known that osmotin induces intracellular signal transduction in yeast. Having a resistance to heat, acidity, and enzymes, it can circulate in the body without being broken down by digestion. This osmotin is known as a homolog of adiponectin found in animals, and adiponectin has been shown in some cases to exhibit possibly anti-inflammatory, antidiabetic, and anti-atherosclerotic properties.

Furthermore, osmotin is widely known for its effects primarily associated with obesity and diabetes, and, in Korean Patent Registration No. 1308232, it is known as a preventive and therapeutic composition for neurological disorders containing osmotin. However, there is no disclosure as of yet for a composition for preventing, ameliorating, or treating Parkinson's disease comprising osmotin protein as effective component as it is described in the present invention.

### DETAILED DESCRIPTION OF THE INVENTION

### TECHNICAL PROBLEMS TO BE SOLVED

The present invention is devised under the circumstances that are described in the above and provided in the present invention is a composition for preventing, ameliorating, or treating Parkinson's disease comprising osmotin protein as effective component. It is found that the osmotin protein, which is the effective component in this composition, has effects of alleviating behavior and motor skill deficits induced by MPTP/α-synuclein, protecting dopaminergic neurons, regulating the expression level of neuroinflammation-related proteins, inhibiting apoptotic cell death induced by MPTP/α-synuclein, alleviating cell damage caused by overexpression of α-synuclein (A53T), reducing the accumulation of α-synuclein caused by activation of AMPK and subsequent autophagy, regulating the dendritic complex structure, increasing the spine density in pyramidal neurons, alleviating the cognitive deficits, and restoring the expression of synaptic markers, and the present invention is completed accordingly.

### TECHNICAL MEANS FOR SOLVING THE PROBLEMS

To achieve the object described in the above, the present invention provides a functional health food composition for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.

The present invention further provides a pharmaceutical composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

The present invention further provides a method of preventing or treating Parkinson's disease including a step of administering to an animal excluding a human a composition comprising osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

The present invention further provides a veterinary composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

The present invention still further provides an animal feed additive for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.

### ADVANTAGEOUS EFFECT OF THE INVENTION

The present invention relates to a composition for preventing, ameliorating, or treating Parkinson's disease comprising osmotin protein as effective component, and the osmotin protein has the effects of alleviating behavior and motor skill deficits induced by MPTP/α-synuclein, protecting dopaminergic neurons, regulating the expression level of neuroinflammation-related proteins, inhibiting apoptotic cell death induced by MPTP/α-synuclein, alleviating cell damage caused by overexpression of α-synuclein (A53T), reducing the accumulation of α-synuclein caused by activation of AMPK and subsequent autophagy, regulating the dendritic complex structure, increasing the spine density in pyramidal neurons, alleviating the cognitive deficits, and restoring the expression of synaptic markers.

### BRIEF DESCRIPTION OF THE DRAWINGS

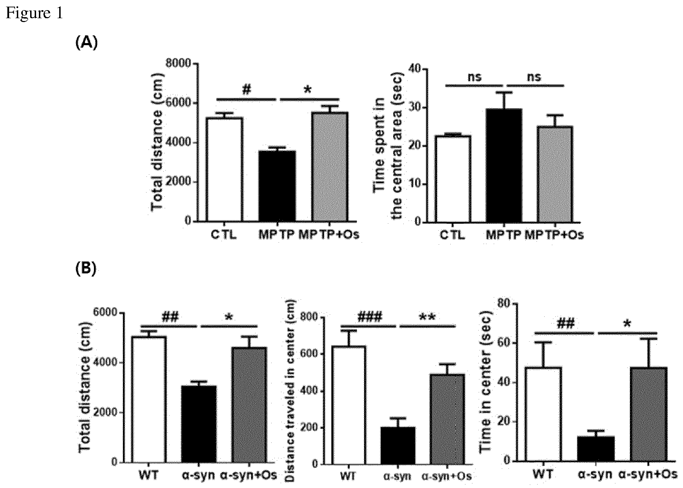

Figure 1 illustrates the results of the open field test (OFT) which has been carried out for determining, after the administration of osmotin protein to (A) MPTP-induced mouse and (B) NSE-hαSyn transgenic mouse, respectively, total travel distance, distance travelled in the central area, and time spent in the central area. #, ##, and ### indicate, in the MPTP group or α-syn group, a statistically significant decrease in the total travel distance, distance travelled in the central area, and time spent in the central area compared to the control group, in which # represents p<0.05, ## represents p<0.01, and ### represents p<0.001. * indicates a statistically significant increase in the total travel distance, distance travelled in the central area, and time spent in the central area for the osmotin administration group of the present invention compared to the MPTP group or α-syn group, with p<0.05. "ns" indicates statistically insignificant changes.

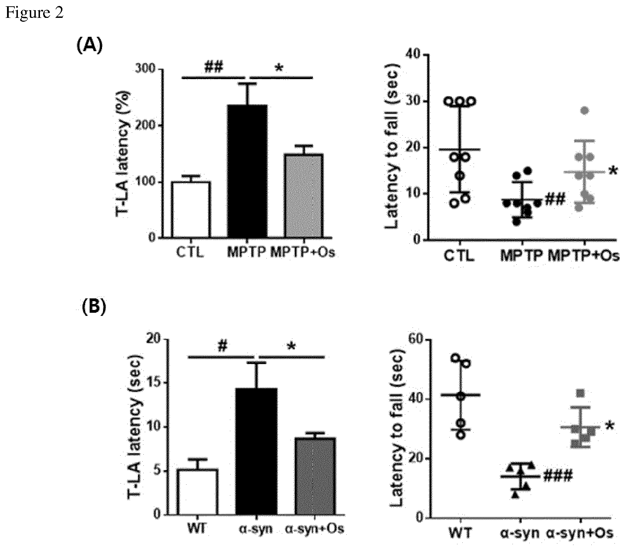

Figure 2 illustrates the results of the pole test and wire-hang test which have been carried out after administering osmotin protein to (A) MPTP-induced mouse and (B) NSE-hαSyn transgenic mouse, respectively. #, ##, and ### indicate, for the MPTP group or α-syn group, a statistically significant increase in the time the animal took to fall from the pole (i.e., latency to fall) and also a significant decrease in hanging time on the wire compared to the control group, in which # represents p<0.05, ## represents p<0.01, and ### represents p<0.001. * indicates, in the osmotin administration group of the present invention, a statistically significant decrease in the time the animal took to fall from the pole and also a significant increase in hanging time on the wire compared to the MPTP group or α-syn group, with p<0.05.

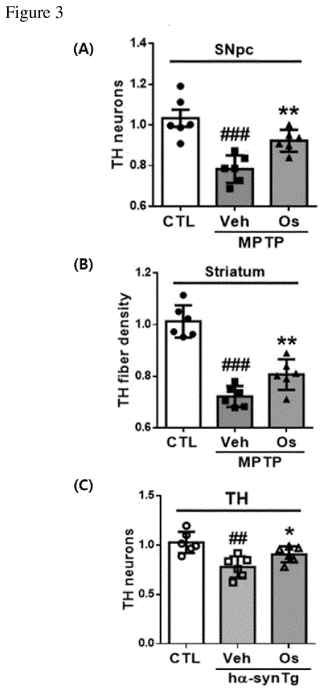

Figure 3 illustrates the results of determining, after administering osmotin protein, (A) the number of TH-positive neurons in the substantia nigra pars compacta (SNpc), (B) the density of TH-positive fibers in the striatum, both examined by TH immunofluorescence staining, and (C) the TH expression level in MPTP/NSE-hαSyn transgenic mouse. ## and ### indicate a statistically significant decrease in the number of TH-positive neurons or TH-positive fiber density in the MPTP-induced group or NSE-hαSyn transgenic mouse group compared to the control group (CTL), in which ## represents p<0.01, and ### represents p<0.001. * and ** indicate a statistically significant increase in the number of TH-positive neurons or TH-positive fiber density for the osmotin administration group of the present invention compared to the MPTP-induced group or NSE-hαSyn transgenic mouse group, in which * represents p<0.05, and ** represents p<0.01.

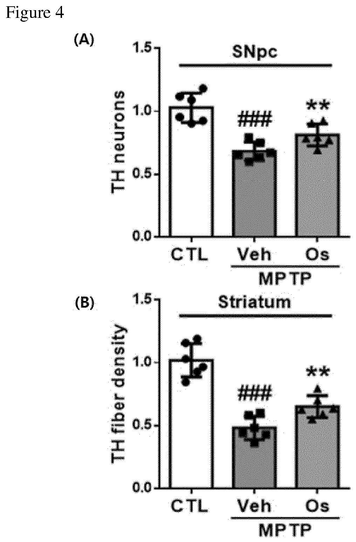

Figure 4 illustrates the results of determining the immune activity of TH-positive neurons in the SNpc and striatum. ### indicates a statistically significant decrease in (A) the number of neurons and (B) TH-positive fiber density in the striatum in the MPTP-induced group compared to the control group (CTL), in which ### represents p<0.001. ** indicates a statistically significant increase in (A) the number of neurons and (B) TH-positive fiber density in the striatum for the osmotin administration group of the present invention compared to the MPTP-induced group, with ** representing p<0.01.

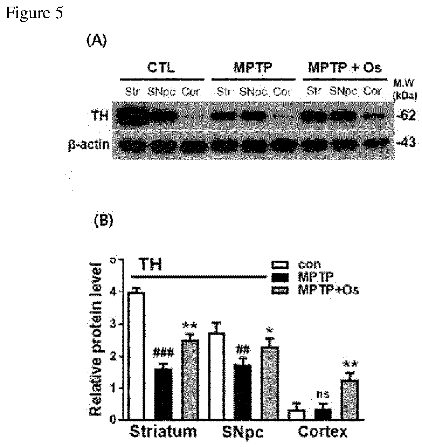

Figure 5 illustrates (A) Western blot image for determining the expression level of TH in the striatum, SNpc, and cortex following the administration of osmotin, and (B) graph in which the Western blot results are quantitatively expressed. ## and ### indicate a decrease in the expression level in the MPTP-induced group compared to the control group (Con), in which ## represents p<0.01, and ### represents p<0.001. * and ** indicate a statistically significant increase in the expression level for the osmotin administration group (MPTP+Os) compared to the MPTP-induced group, in which * represents p<0.05 and ** represents p<0.01.

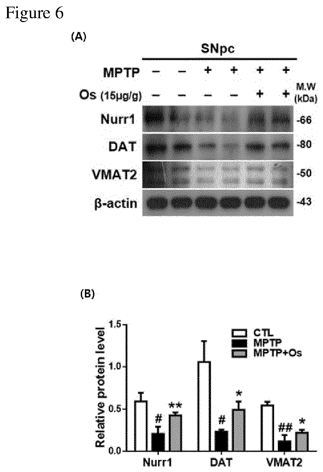

Figure 6 illustrates (A) Western blot image for determining the expression level of Nurr1, DAT, and VMAT2 in the SNpc following the administration of osmotin to MPTP/NSE-hαSyn transgenic mouse, and (B) graph in which the Western blot results are quantitatively expressed. # and ## indicate a decrease in the expression level in the MPTP-induced group compared to the control group (Con), in which # represents p<0.05 and ## represents p<0.01. * and ** indicate a statistically significant increase in the expression level for the osmotin administration group (MPTP+Os) compared to the MPTP-induced group, in which * represents p<0.05, and ** represents p<0.01.

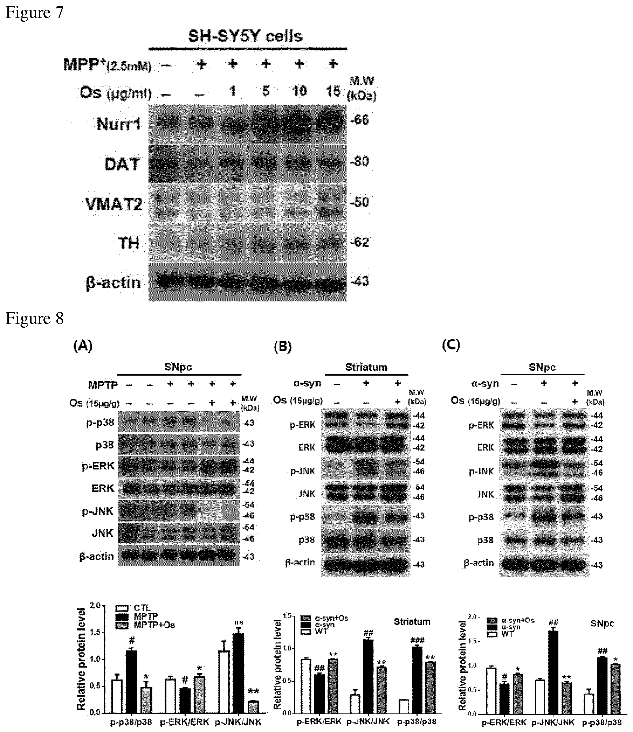

Figure 7 shows the Western blot results for determining the expression level of Nurr1, DAT, VMAT2, and TH in SH-SY5Y cells, which have been previously induced with MPP⁺, after the induction with MPTP and treatment with osmotin.
Figure 8 illustrates the results of determining the expression level of p-p38/p38, p-ERK/ERK, and p-JNK/JNK proteins in the SNpc and striatum following the administration of osmotin of the present invention to MPTP/NSE-hαSyn transgenic mouse. # and ## indicate a statistically significant increase or decrease in the expression level in the MPTP group or αSyn group compared to the control groups (CTL, WT), in which # represents p<0.05, and ## represents p<0.01. * and ** indicate a statistically significant decrease or increase in the expression level for the osmotin administration group of the present invention compared to the MPTP group or αSyn group, in which * represents p<0.05, and ** represents p<0.01.

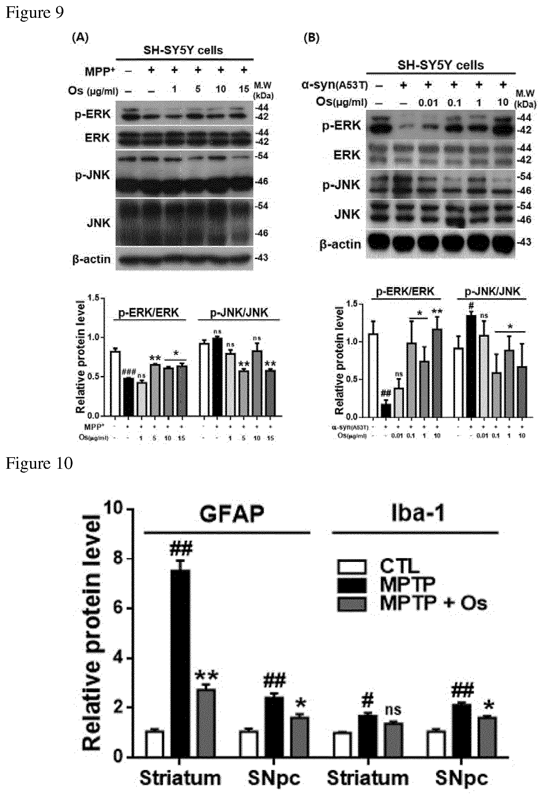

Figure 9 illustrates the results of determining the expression level of p-ERK/ERK and p-JNK/JNK proteins in SH-SY5Y cells, which have been previously induced with MPP⁺/α-synuclein (A53T), following the administration of osmotin protein. #, ##, and ### indicate a statistically significant increase or decrease in the expression level in the MPP⁺ group or αSyn group compared to the control groups (CTL, WT), in which # represents p<0.05, ## represents p<0.01, and ### represents p<0.001. * and ** indicate a statistically significant decrease or increase in the expression level for the osmotin administration group of the present invention compared to the MPP⁺ group or αSyn group, in which * represents p<0.05, and ** represents p<0.01. "ns" indicates statistically insignificant changes.
Figure 10 illustrates the changes in GFAP and Iba-1 expression level in the striatum and SNpc of MPTP/NSE-hαSyn transgenic mouse following the administration of osmotin. # and ## indicate a statistically significant increase in GFAP and Iba-1 expression level in the MPTP group compared to the control group, in which # represents p<0.05, and ## represents p<0.01. * and ** indicate a statistically significant decrease in GFAP and Iba-1 expression level for the osmotin administration group of the present invention compared to the MPTP group, in which * represents p<0.05, and ** represents p<0.01. "ns" indicates statistically insignificant changes.

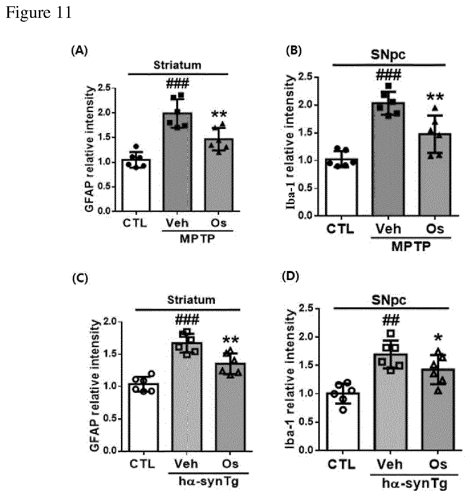

Figure 11 illustrates the results of immunofluorescence analysis of the expression of GFAP and Iba-1 in (A) the striatum and (B) SNpc region of MPTP/NSE-hαSyn transgenic mouse following the administration of osmotin. ## and ### indicate a statistically significant increase in GFAP and Iba-1 expression level in the MPTP group compared to the control group, in which ## represents p<0.01, and ### represents p<0.001. * and ** indicate a statistically significant decrease in GFAP and Iba-1 expression level for the osmotin administration group of the present invention compared to the MPTP group, in which * represents p<0.05, and ** represents p<0.01.

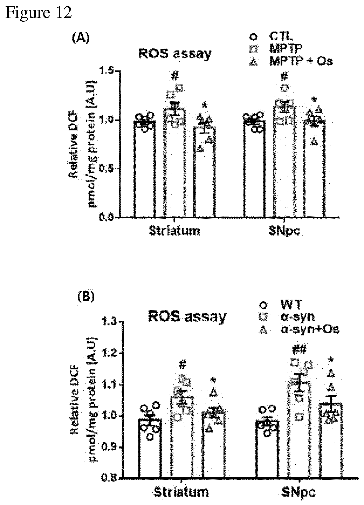

Figure 12 illustrates the results of the ROS assay for the striatum and SNpc following the administration of osmotin. # and ## indicate a statistically significant increase in the relative DCF (i.e., staining agent for ROS measurement) levels in the MPTP group compared to the control group, in which # represents p<0.05, and ## represents p<0.01. * indicates a statistically significant decrease in the relative DCF levels for the osmotin administration group of the present invention compared to the MPTP group, in which * represents p<0.05.

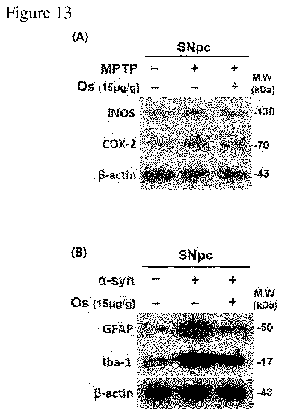

Figure 13 illustrates the Western blot results for determining the expression level of iNOS and COX-2 in the SNpc region of MPTP/NSE-hαSyn transgenic mouse following the administration of osmotin.

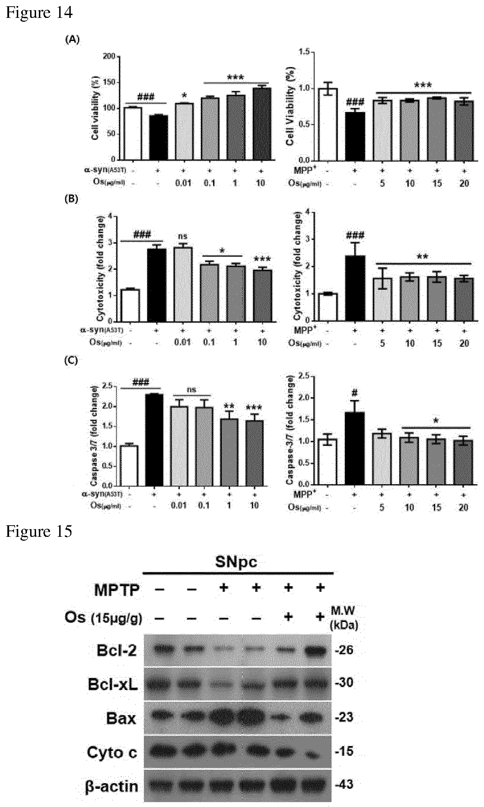

Figure 14 illustrates the analysis results of (A) cell viability, (B) cytotoxicity, and (C) caspase-3/7 activity following the administration of osmotin to MPTP/α-synuclein-induced mouse group. # and ### indicate a statistically significant decrease in cell viability, an increase in cytotoxicity, and an increase in caspase-3/7 activity in the MPTP/α-synuclein-induced group compared to the control group, in which # represents p<0.05, and ### represents p<0.001. *, **, and *** indicate a statistically significant increase in cell viability, a decrease in cytotoxicity, and a decrease in caspase-3/7 activity in the osmotin administration group of the present invention compared to the MPTP/α-synuclein-induced group, in which * represents p<0.05, ** represents p<0.001, and *** represents p<0.001.
Figure 15 illustrates the Western blot results for determining the expression level of Bcl-2, Bcl-xL, Bax, and Cytochrome-c (Cyto c), which are involved in MPTP/MPP⁺-induced neuronal cell death, following the treatment with osmotin protein.

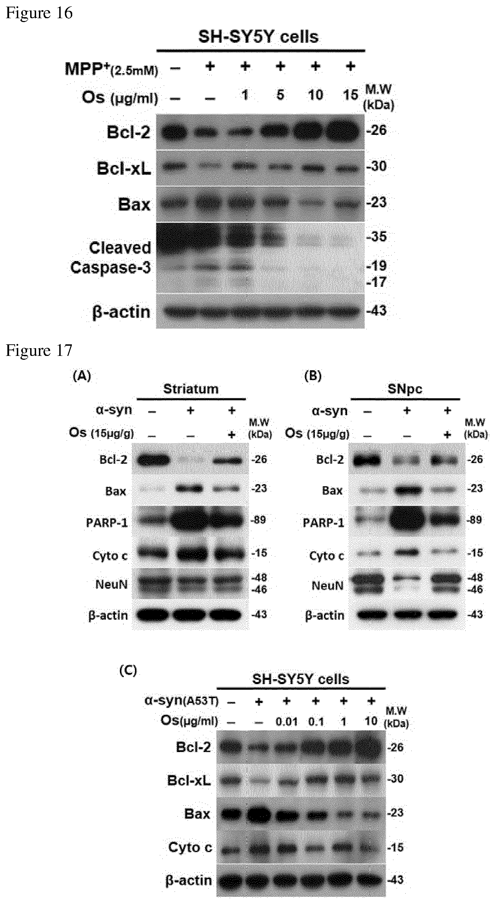

Figure 16 illustrates the Western blot results for determining the expression level of Bcl-2, Bcl-xL, Bax, and cleaved caspase-3 in SH-SY5Y cells, which have been previously induced by MPP⁺, following the treatment with osmotin.
Figure 17 illustrates the Western blot results for determining the expression level of Bcl-2, Bax, PARP-1, Cytochrome c, and NeuN, which are involved in cell death in (A) the striatum and (B) SNpc region of NSE-hαSyn transgenic mouse following the administration of osmotin protein, and also the expression level of Bcl-2, Bcl-xL, Bax, cytochrome c, and NeuN, which are involved in cell death in (C) the SH-SY5Y cells, which have been previously induced by α-synuclein (A53T), following the administration of osmotin protein.

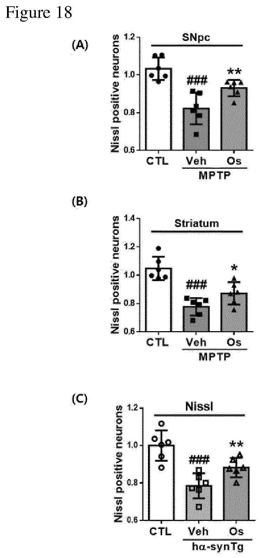

Figure 18 illustrates the results of determining (A and B) the neuron count in the striatum and SNpc of MPTP/α-synuclein-induced group, and (C) Nissl-stained neuron count in the PTP/NSE-hαSyn transgenic mouse. ### indicates a statistically significant decrease in neuron count in the MPTP group compared to the control group (p<0.001). * and ** indicate a statistically significant increase in neuron count in the osmotin administration group of the present invention compared to the MPTP group, with * indicating p<0.05 and ** indicating p<0.01.

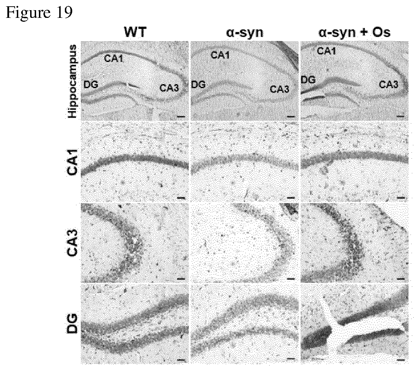

Figure 19 illustrates the Nissl staining results for the CA1, CA3, and DG regions in NSE-hαSyn transgenic mouse following the administration of osmotin.

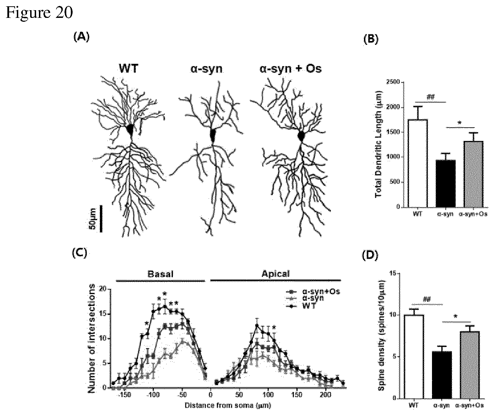

Figure 20 illustrates the results of Golgi staining for determining (A) the structure of the dendritic cell complex, (B) the dendritic length of NSE-hαSyn mice, (C) the number of intersections in the basal and apical regions of the brain of NSE-hαSyn mice, and (D) spine density in NSE-hαSyn mice. ## indicates a statistically significant decrease in dendrite length, number of intersections, or spine density in the NSE-hαSyn mouse group compared to the control group (p<0.01). * indicates a statistically significant increase in dendrite length, number of intersections, or spine density in the osmotin administration group of the present invention compared to the NSE-hαSyn mouse group (p<0.05).

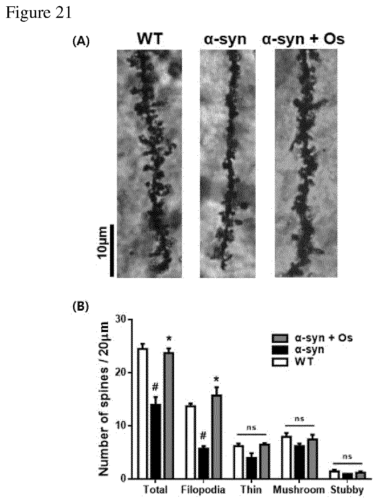

Figure 21 illustrates the results of determining filopodia spines in the NSE-hαSyn mouse group, showing (A) images of filopodia spines and (B) number of filopodia spines. # indicates a statistically significant decrease in the number of filopodia spines in the NSE-hαSyn mouse group compared to the control group (p<0.05), while * indicates a statistically significant increase in the number of filopodia spine in the osmotin administration group of the present invention compared to the NSE-hαSyn mouse group (p<0.05). "ns" denotes statistically insignificant changes.

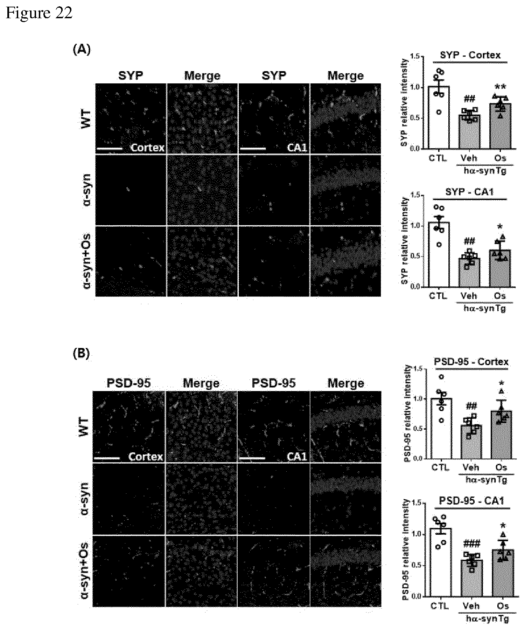

Figure 22 illustrates the immunofluorescence analysis results for (A) synaptophysin (SYP) and (B) postsynaptic density protein 95 (PSD-95) in the cortex and CA1 following the administration of osmotin. ## and ### indicate a statistically significant decrease in the expression level of SYP and PSD-95 in the NSE-hαSyn mouse group compared to the control group (p<0.01 for ## and p<0.001 for ###, respectively). * and ** indicate a statistically significant increase in the expression level of SYP and PSD-95 in the osmotin administration group of the present invention compared to the NSE-hαSyn mouse group (p<0.05 for * and p<0.01 for **, respectively).

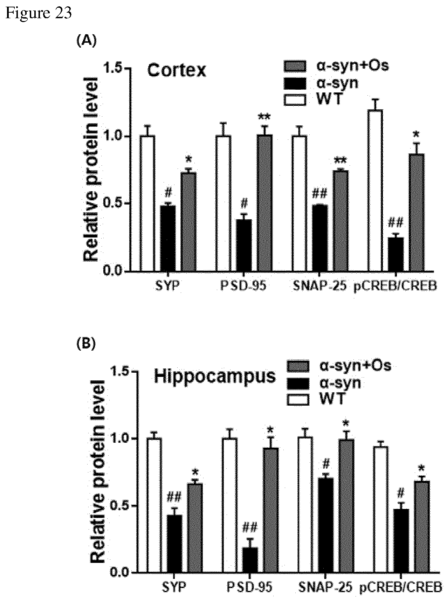

Figure 23 illustrates the Western blot results for SYP, PSD-95, SNAP-25, and pCREB/CREB in (A) the cortex and (B) hippocampus following the administration of osmotin. ## and ### indicate a statistically significant decrease in the expression level of SYP, PSD-95, SNAP-25, and pCREB/CREB in the NSE-hαSyn mouse group compared to the control group (p<0.01 for # and p<0.001 for ##, respectively). * and ** indicate a statistically significant increase in the expression level of SYP, PSD-95, SNAP-25, and pCREB/CREB in the osmotin administration group of the present invention compared to the NSE-hαSyn mouse group (p<0.05 for * and p<0.01 for **, respectively).

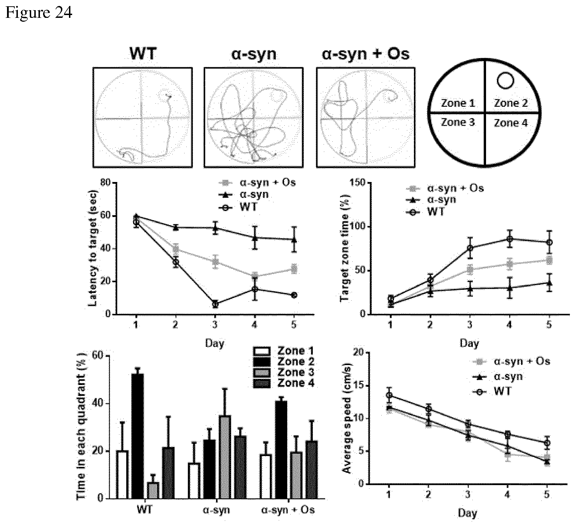

Figure 24 illustrates the results of determining the effects of osmotin on behavioral changes induced by α-synuclein.

### BEST MODE (S) FOR CARRYING OUT THE INVENTION

The present invention relates to a functional health food composition for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.

In the present invention, osmotin is a protein abundantly present in mature fruits such as grapes, and it is a protein consisting of approximately 150 to 250 amino acids depending on the organism.

While it is preferred for the osmotin protein to be contained in the composition in an amount ranging from 0.1 to 100% by weight relative to the total weight of the composition, it is not limited thereto.

The osmotin protein described above can regulate the expression level of one or more genes or proteins selected from Nurr1 (Nuclear receptor related 1 protein), VMAT2 (vesicular monoamine transporter 2), DAT (dopamine transporter), TH (tyrosine hydroxylase), p-p38, p-JNK (c-Jun N-terminal kinase), and p-ERK (extracellular signal-regulated kinase).

The functional health food composition can be produced as any one food product selected from beverage, pill, tablet, capsule, or powder formulation. Alternatively, it can also be produced by adding to other food products or food ingredients and can be produced according to conventional methods.

Examples of the food product to which the composition of the present invention can be added include meat, sausage, chocolate, candies, snacks, biscuits, pizza, ramen, other noodles, gums, dairy products including ice cream, various kinds of soup, beverage, tea, drink, alcohol beverage, and vitamin complex, and all functional health food products in general sense are included therein.

The functional health food composition may further comprise various nutritional supplements, a vitamin, a mineral (i.e., electrolyte), a synthetic or natural flavor, a coloring agent, an enhancer (i.e., cheese, chocolate, etc.), pectinic acid and a salt thereof, alginic acid and a salt thereof, an organic acid, a protective colloidal thickening agent, a pH adjusting agent, a stabilizer, a preservative, glycerin, alcohol, and a carbonating agent used for carbonated beverage. Other than those, fruit pulp for producing natural fruit juice or vegetable drink may be additionally comprised. Those components may be used either independently or in combination thereof, and various flavorings or natural carbohydrates can be also comprised as additional ingredients. The natural carbohydrates may include monosaccharides such as glucose and fructose, disaccharides such as maltose and sucrose, and polysaccharides such as dextrin and cyclodextrin, as well as sugar alcohols such as xylitol, sorbitol, and erythritol. As sweeteners, natural sweeteners such as taumatin and stevia extracts, or synthetic sweeteners such as saccharin and aspartame, can be used.

The present invention further relates to a pharmaceutical composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

In addition to the effective component of the pharmaceutical composition mentioned above, the pharmaceutical composition may further comprise one or more carriers selected from pharmaceutically acceptable saline, sterile water, Ringer's solution, buffered saline, dextrose solution, maltodextrin solution, glycerol, ethanol, lactose, dextrose, sucrose, sorbitol, mannitol, starch, acacia gum, calcium phosphate, alginate, gelatin, calcium silicate, microcrystalline cellulose, polyvinyl pyrrolidone, cellulose, syrup, methyl cellulose, methylhydroxybenzoate, propylhydroxybenzoate, talc, magnesium stearate, and mineral oil. Additionally, besides the effective component, the pharmaceutical composition may also comprise one or more aiding agent selected from pharmaceutically acceptable antioxidant, buffering agent, bacteriostatic agent, diluent, surfactant, binder, lubricant, wetting agent, sweetener, flavoring agent, emulsifier, suspending agent, and preservative.

The pharmaceutical composition can be administered in form of oral formulation or parenteral formulation according to conventional methods. In the case of preparing formulation, it is typically prepared using diluents or excipients commonly used in the art, such as fillers, bulking agents, binders, wetting agents, disintegrants, surfactants, and the like. Examples of the solid formulation for oral administration include tablet, pill, powder preparation, granule, and capsule. These solid formulations are typically prepared by blending at least one excipient, such as starch, calcium carbonate, sucrose, lactose, or gelatin with the aforementioned composition.

Lubricants like magnesium stearate or talc may also be used in addition to the simple excipients. Examples of the liquid formulation for oral administration include suspensions, solutions, emulsions, syrups, and the like. These liquid formulations may comprise, in addition to simple diluents like water or liquid paraffin, various excipients such as wetting agents, sweeteners, flavorings, preservatives, and the like. Examples of the formulation for parenteral administration include sterile solutions, non-aqueous solvents, suspensions, emulsion, freeze-dried formulations, and suppositories. In addition, as the non-aqueous solvents or suspension, propylene glycol, polyethylene glycol, plant-based oils like olive oil, injectable esters like ethyl oleate, and the like may be used. Examples of the base used for suppositories include Witepsol, Macrogol, Tween 61, cacao fat, laurin fat, glycerol gelatin, and the like.

The present invention further relates to a method of preventing or treating Parkinson's disease including a step of administering to an animal excluding a human a composition comprising osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

The animal may include a human, or it can be an animal other than a human.

The present invention further provides a veterinary composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

The veterinary composition of the present invention may further comprise appropriate excipients and diluents according to conventional methods. Examples of the excipients and diluents that can be comprised in the veterinary composition of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, amorphous cellulose, polyvinylpyrrolidone, water, methylhydroxybenzoate, propylhydroxybenzoate, talc, magnesium stearate, cetanol, stearyl alcohol, liquid paraffin, sorbitan monostearate, polysorbate 60, methylparaben, propylparaben, and mineral oil. The veterinary composition according to the present invention may additionally include fillers, anticoagulants, lubricants, wetting agents, flavorings, emulsifiers, preservatives, and the like. The veterinary composition according to the present invention can be formulated using the methods that are well known in the industry to provide rapid, sustained, or delayed release of the active ingredient after administration to animals. The formulation can take various forms such as powder, granule, tablet, capsule, suspension, emulsion, solution, syrup, aerosol, soft or hard gelatin capsule, suppository, sterile injectable solution, or sterile topical preparation.

The effective amount of the veterinary composition according to the present invention can be suitably selected according to the individual characteristics of the animal. Specifically, the effective amount can be determined based on factors such as the severity of the disease or condition, the age, weight, health status, or gender of the animal, sensitivity to the effective component of the present invention, the route of administration, the duration of administration, other composition used in combination or simultaneously with the composition of the present invention, and other factors that are well known in the fields of physiology and veterinary medicine.

The present invention still further provides an animal feed additive for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.

The animal feed additive of the present invention corresponds to a supplementary animal feed prescribed in the animal feed management regulations. In the present invention, the term 'animal feed' may refer to any natural or artificial prescribed feed, single feed, or the components of such single feed, which animals eat, ingest, and digest. The types of the animal feed are not particularly limited and can include the feeds that are commonly used in the relevant technical field. Non-limiting examples of such feeds include plant-based feeds such as grains, root vegetables, by-products of food processing, poultry, fibers, pharmaceutical by-products, oils, starches, gourd proteins, and cereal by-products; and animal-based feeds such as proteins, minerals, oils, mineral substances, fats, single-cell proteins, animal plankton, or food. These can be used either alone or in a mixture of two or more of them.

Hereinbelow, the present invention is explained in greater detail in view of Examples. However, the following Examples are given only for more specific explanation of the present invention and it is evident to a person who has common knowledge in the pertinent art that the scope of the present invention is not limited by them.

### EXAMPLES

### [Test Animals]

As an animal for inducing Parkinson's disease via MPTP (1-methyl-4-phenyl-1,2,3,6-tetrahydropyridine) treatment, male wild-type C57BL/6J mice (approximately 25 to 28 g, 8 weeks old) were purchased from Jackson Laboratory (Bar Harbor, ME, USA). C57BL/6-Tg (NSE-hαSyn) Korl mice were obtained from the National Institute of Food and Drug Safety Evaluation (NIFDS, Cheongju, South Korea). The test animals were acclimated for 1 week under a 12-h dark/light cycle at 21 to 23°C with 60±10% humidity and provided food and water *ad libitum.*

MPTP (Sigma-Aldrich, MO, USA) was prepared in sterile distilled water and administered intraperitoneally (i.p.) at a dose of 30 mg/kg for 5 consecutive days, according to the guidelines which have been previously established.

C57BL/6J mice were randomly allocated into the following three groups, i.e., Control (CTL), MPTP-treated group, and MPTP plus osmotin-treated group (MPTP+Os), in which 12 mice were allocated for each group.

C57BL/6-Tg (NSE-hαSyn) Korl mice were allocated into the following three groups, i.e., wild-type group (WT), control group (NSE-hαSyn; α-syn), and NSE-hαSyn plus osmotin-treated group (α-syn+Os), in which 12 mice were allocated for each group.

With regard to the treatment with osmotin, purified osmotin was administered at a dose of 15 mg/kg in saline.

### [Cell Culture]

Human neuroblastoma SH-SY5Y cells were purchased from Korea Cell Line Bank (KCLB, South Korea) and cultured in Dulbecco's modified Eagle's medium (DMEM; Gibco, NY, USA) containing 1% antibiotic-antimycotic solution and 10% fetal bovine serum incubated at 37°C with 5% CO₂. The mHippoE-14 embryonic mouse hippocampal cell line (Cedarlane, Canada) was derived at embryo day 14 and cultured in DMEM without sodium pyruvate. SH-SY5Y cells were treated with MPP⁺ (Sigma-Aldrich, MO, USA). The EGFP-alpha-synuclein-A53T plasmid was obtained from David Rubinsztein (Addgene #40823). Adiponectin receptor (AdipoR1) was knocked out by commercial AdipoR1 CRISPR/Cas9-KO Plasmid (Santa Cruz, CA, USA).

The insertion of the puromycin gene as a selection marker was performed using a AdipoR1 HDR plasmid (Santa Cruz, CA, USA). Cells were transfected using the abovementioned plasmids with Lipofectamine 3000 (Invitrogen, CA, USA). The cells were then cultured, and the medium was removed 24 hours before selection. A pure and stable pool of knockout cell lines was obtained by using 2.5 µg/ml puromycin which has been added to the culture medium as a selection marker.

### Example 1. Determination of effect of ameliorating MPTP/α-synuclein-induced behavior and motor skill deficits by administration of osmotin protein

To investigate the protective effects of osmotin against motor skill deficits shown in MPTP-induced and NSE-hαSyn transgenic mice, behavioral tests were carried out regarding the motor skill impairments.

Specifically, in the open-field test (OFT), the MPTP/NSE-hαSyn transgenic mice showed a decrease in the total travel distance. However, in the group receiving osmotin administration, there was a significant increase in the total distance traveled by the mice compared to the MPTP/NSE-hαSyn transgenic mice (Figure 1).

Furthermore, to evaluate the effects of osmotin protein on physical activity and neuromuscular strength of the mice, a pole test and wire-hang test were carried out.

As a result, in the pole test, the mice of the MPTP/α-synuclein group showed a markedly longer total time (T-LA) to return to the floor compared with that of the control and WT groups. On the other hand, in the group administered with osmotin protein of the present invention, there was a statistically significant decrease in the total time (T-LA) to return to the floor.

In the wire-hang test, the MPTP/α-synuclein group showed a decrease in hang time, i.e., latency to fall, compared to the control group and the wild type group. On the other hand, in the group administered with osmotin protein of the present invention, there was a statistically significant increase in the latency to fall. Based on these results, it was recognized that a loss of neuromuscular strength is caused in the MPTP/NSE-hαSyn transgenic mice, but, due to the neuroprotective effect, osmotin can reduce remarkably the motor skill deficits triggered in the MPTP/NSE-hαSyn transgenic mice (Figure 2).

### Example 2. Determination of regulation of expression level of Nurr1 (Nuclear receptor related 1 protein), VMAT2 (vesicular monoamine transporter 2), DAT (dopamine transporter), and TH (tyrosine hydroxylase) by administration of osmotin protein

To determine whether osmotin protects the mice against the MPTP/α-synuclein-induced loss of dopaminergic neurons, the number of TH-positive neurons in the SNpc and the striatal TH-positive fiber density were measured by TH fluorescent staining. The TH level was significantly reduced in the MPTP/NSE-hαSyn transgenic mice compared to that in the mice of the control group and wild type group (WT). However, it was also found that the reduction in TH level can be markedly restored with the administration of osmotin protein (Figure 3).

Moreover, immunoreactivity of TH-positive neurons was examined in the SNpc and striatum of the mice. It was found that there is a significant decrease in TH-positive neurons in the MPTP-induced mice compared with the number in the control group. However, with osmotin protein, a significant increase in TH-positive neurons was shown from the SNpc and striatum of the MPTP-induced mice (Figure 4).

Similar to above, changes in the TH expression level were examined by using Western blot analysis. As a result, it was found that the TH expression in the striatum and SNpc was significantly lower in the MPTP/NSE-hαSyn transgenic mice compared to that in the mice of the control group and WT. it was also found that osmotin can efficiently enhance the expression level of TH (Figure 5).

Furthermore, the Nurr1, VMAT2, and DAT expression levels in the SNpc and striatum of MPTP/NSE-hαSyn transgenic mice *in vivo* models were found to be significantly upregulated with the administration of osmotin (Figure 6).

Meanwhile, it was also found that the expression levels of Nurr1, DAT, VMAT2, and TH are lower in in the MPP⁺-induced SH-SY5Y cells. On the contrary, in the group treated with osmotin protein, there were significantly upregulated expression levels of Nurr1, DAT, VMAT2, and TH in a dose-dependent manner (Figure 7).

### Example 3. Determination of effects of osmotin protein on regulating expression levels of p-p38, p-JNK, and p-ERK and neuroinflammation

To determine the effects of osmotin protein on p38, JNK and ERK phosphorylation, the expression levels of p-p38, p-JNK, and p-ERK in MPTP/α-synuclein-induced *in vivo* and *in vitro* models were analyzed.

As a result, it was found that there is an increased expression levels of p-p38 and p-JNK and a decreased expression level of p-ERK in the MPTP/NSE-hαSyn transgenic mice. On the other hand, in the group treated with osmotin protein, there was a significantly decreased expression levels of p-p38 and p-JNK and increased expression level of p-ERK compared to the MPTP/NSE-hαSyn transgenic mice (Figure 8).

Furthermore, as a result of evaluating the effect of osmotin against MPP⁺/α-synuclein (A53T)-induced SH-SY5Y cells, it was shown that the treatment of osmotin protein significantly increased, in dose-dependent manner, the expression level of p-ERK/ERK, which has been decreased in MPP⁺/α-synuclein-induced SH-SY5Y cells. In addition, the expression level of p-JNK/JNK was higher in the MPP⁺/α-synuclein (A53T) induced group, but it becomes lower in dose-dependent manner in the group treated with osmotin protein of the present invention (Figure 9).

Furthermore, to evaluate the effects on neuroinflammatory response, the effect of osmotin against activated astrocytes (GFAP) and microglial cells (Iba-1) was examined.

As a result, it was found that GFAP and Iba-1 expression levels were significantly increased in the striatum and SNpc of the MPTP/NSE-hαSyn transgenic mice, but such increase was significantly reduced with the administration of osmotin (Figure 10).

Furthermore, the results of the immunofluorescence studies support that osmotin can significantly reduce the expression of GFAP and Iba-1 in the striatum and SNpc regions of the MPTP/NSE-hαSyn transgenic mice (Figure 11).

To determine the possible role of oxidative stress in relation to the activation of astrocytes and microglia, an ROS quantitative assay was carried out. As a result, it was found that ROS were significantly reduced in the osmotin administration group compared to their level in the MPTP/NSE-hαSyn transgenic mice (Figure 12).

Still furthermore, when the expression of iNOS and COX-2 was examined, it was found that there is an increase in the expression of iNOS and COX-2 in the mice which had been induced with MPTP/NSE-hαSyn. However, compared to those mice, a decrease in the expression of iNOS and COX-2 was shown from the group administered with osmotin (Figure 13).

### Example 4. Determination of regulatory effect of osmotin protein on MPTP/α-synuclein-induced cell apoptosis in animal and cellular models

To evaluate the effects of osmotin against MPP⁺/α-synuclein (A53T)-induced toxicity, cell viability, cytotoxicity, and caspase-3/7 activity analyses were carried out by using ApoTox-Glo Triplex assays.

As a result, it was found that MPP⁺ treatment reduced the cell viability and increased the cytotoxicity and caspase-3/7 activity. On the other hand, when treated with osmotin protein of the present invention, higher cell viability with lower cytotoxicity and lower caspase-3/7 activity was shown.

The effects of osmotin against α-synuclein (A53T)-induced decrease in cell viability and effects on cytotoxicity and caspase-3/7 activities were also investigated. As a result, it was found that osmotin exhibited significant effects on cell viability, cytotoxicity and caspase-3/7 activity in a dose-dependent manner (Figure 14).

It is well known that mitochondria regulate the processes of cell death and degeneration. To investigate whether osmotin can inhibit the MPTP/MPP⁺-induced neuronal cell death, analysis of pro-/anti-apoptotic markers was carried out using Western blots.

According to the result, it was found that MPTP significantly increases the level of Bax and significantly decreases Bcl-2 and Bcl-xL levels. In contrast, osmotin protein reduced the secretion amounts of Bax and cytochrome c but increased the expression of Bcl-2 and Bcl-xL (Figure 15).

Furthermore, the expression of mitochondrial apoptotic markers in MPP⁺-induced SH-SY5Y cells was examined. As a result, it was found that MPP⁺ significantly decreased Bcl-2 and Bcl-xL levels and increased expression levels of Bax and cleaved caspase-3. On the other hand, osmotin reduced the expression levels of pro-apoptotic markers, such as Bax and cleaved caspase-3, and increased anti-apoptotic markers, such as Bcl-2 and Bcl-xL, in a concentration-dependent manner (Figure 16).

It was also found that osmotin exhibits anti-apoptotic effects against apoptotic cell death in the striatum and SNpc of NSE-hαSyn transgenic mice, and, in a significant way, osmotin regulated the expression levels of Bcl-2, Bax, cytochrome c, and PARP-1. The NeuN expression level was lower in the NSE-hαSyn, but the NeuN expression level was restored by osmotin protein (Figures 17A and 17B).

The *in vivo* effects of osmotin against apoptosis in NSE-hαSyn mice were also determined *in vitro* in α-synuclein (A53T)-induced cells. It was found that the overexpression of α-synuclein (A53T) significantly decreased Bcl-2 and Bcl-xL expression levels but increased Bax and cytochrome c expression levels. On the other hand, osmotin markedly increased the levels of Bcl-2 and Bcl-xL and reduced the expression levels of Bax and cytochrome c in a concentration-dependent manner (Figure 17C).

The MPTP/α-synuclein-induced neurotoxic effects in the striatum and SNpc were analyzed by Nissl staining. As a result, it was found that there is a significant reduction in Nissl-stained neurons in the MPTP/NSE-hαSyn mice compared to the number of the neurons in the control group (i.e., Control or WT). On the other hand, in the group administered with osmotin protein, a significant restoration of the Nissl-stained neurons was obtained compared to MPTP/NSE-hαSyn mouse group (Figure 18).

### Example 5. Determination of effect of osmotin protein for regulating dendritic complexity and structure and increasing spine density in pyramidal neurons

To analyze the above-mentioned effects by osmotin treatment, Nissl staining was carried out for the subjects of the test group. The number of Nissl-stained neurons in the CA1, CA3, and DG regions of the NSE-hαSyn mice was significantly lower than that of WT mice, but this reduction was significantly upregulated with osmotin administration (Figure 19).

To analyze the effects of osmotin protein on spine morphology as altered by α-synuclein, Golgi staining was carried out so that the dendritic structure, complexity, and length in hippocampal CA1 pyramidal neurons can be determined. As a result, it was found that there is a significant decrease in total dendritic length in the NSE-hαSyn mice compared with that of the dendrites in the WT mice. On the contrary, in the group treated with osmotin protein, there was an increase in the total dendritic length compared with that of the dendrites in the NSE-hαSyn mice. Additionally, it was also found that the dendritic complexity was reduced in the NSE-hαSyn mice compared with that in the WT mice, while osmotin protein significantly increased the complexity in the basal and apical regions of NSE-hαSyn mouse brains.

It was also found that the spine density and the total number of spines in the NSE-hαSyn transgenic mice were significantly decreased compared with the density and number in the WT mice, but they were remarkably upregulated upon the administration of osmotin protein (Figure 20).

In particular, the number of filopodia-like spines in the NSE-hαSyn group was significantly decreased compared with that in the WT group, while, in the group administered with osmotin protein, there was a significant increase in the spine density, total number of spines, and filopodia-like spines compared with those in the NSE-hαSyn group (Figure 21).

### Example 6. Determination of effects of osmotin protein for alleviating cognitive deficits and rescuing synaptic marker expression in NSE-hαSyn mice

To analyze the protective effects of osmotin against synaptic dysfunction in the Parkinson's disease model, immunofluorescence analyses of synaptophysin (SYP) and postsynaptic density protein-95 (PSD-95) were carried out. As a result of the immunofluorescence analyses, it was found that there is a significant decline in the expression of SYP and PSD-95, which are present in the frontal cortex and CA1 region of the NSE-hαSyn transgenic mice, compared to that in the WT mice. The downregulation of PSD-95 and SYP was markedly reversed, i.e., upregulated, when osmotin is administered (Figure 22).

The immunofluorescence results were further confirmed by Western blot analyses. The expressions of SYP, PSD-95, and SNAP-25, as well as the phosphorylation of the memory-associated protein CREB, were significantly reduced in the cortex and hippocampus of the NSE-hαSyn mice compared with the levels in the WT mice. However, in the group administered with osmotin protein, the expressions of SYP, PSD-95, and SNAP-25 and the phosphorylation of CREB in NSE-hαSyn mice were significantly upregulated (Figure 23).

The MWM test was conducted to analyze the effects of osmotin protein against α-synuclein-induced behavioral changes in NSE-hαSyn mice. As a result of the test, it was found that both the latency to reach the platform and latency to reach the target quadrant were significantly improved after the administration of osmotin. The time spent in the target zone was shorter in the NSE-hαSyn mice compared with the WT mice, while the percentage of time spent in the quadrant (zone 2) where the platform was placed was significantly smaller in the NSE-hαSyn mice compared with the WT mice. On the contrary, when osmotin protein is administered to NSE-hαSyn mice, higher frequency of time spent at target zone and longer time spent in the quadrant were shown (Figure 24).

### [Statistical Analysis]

The data were analyzed using GraphPad Prism 6 software. Statistical data are presented as the mean ± standard deviation of the mean (SEM) based on at least three independent tests with 12 mice per group. For the morphological analysis, three images obtained from at least 3 tests with similar results were considered. The significance of the differences was determined by one or two-way analysis of variance (ANOVA) followed by Student's t-tests. As a result of the evaluations, results with p<0.05 were considered significant.

## Claims

1. A functional health food composition for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.

2. The functional health food composition according to Claim 1, wherein the osmotin protein is contained at 0.1 to 100% by weight relative to total weight of the composition.

3. The functional health food according to Claim 1, wherein the composition is prepared in any one formulation selected from beverage, pill, tablet, capsule, and powder formulation.

4. The functional health food composition according to Claim 1, wherein the osmotic protein regulates expression level of one or more genes or proteins selected from Nurr1 (Nuclear receptor related 1 protein), VMAT2 (vesicular monoamine transporter 2), DAT (dopamine transporter), TH (tyrosine hydroxylase), p-p38, p-JNK (c-Jun N-terminal kinase) and p-ERK (extracellular signal-regulated kinase).

5. A pharmaceutical composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

6. A method of preventing or treating Parkinson's disease including administering to an animal excluding a human a composition comprising osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

7. A veterinary composition for preventing or treating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or treating Parkinson's disease.

8. An animal feed additive for preventing or ameliorating Parkinson's disease comprising, as effective component, osmotin protein selected from the following: (a) osmotin protein having the amino acid sequence of SEQ ID NO: 1; or (b) osmotin protein derived from above (a) with one or more amino acid residues substituted, deleted, or inserted within the amino acid sequence of SEQ ID NO: 1, and capable of preventing or ameliorating Parkinson's disease.
